(19) Europäisches Patentamt
European Patent Office
Office européen des brevets

(11) EP 1 896 139 B1

(12) **EUROPEAN PATENT SPECIFICATION**

(45) Date of publication and mention of the grant of the patent:
**31.03.2010 Bulletin 2010/13**

(21) Application number: **06732643.9**

(22) Date of filing: **12.05.2006**

(51) Int Cl.:
***A61Q 5/04** (2006.01)* ***A61Q 5/10** (2006.01)*
***A61K 8/49** (2006.01)* ***A61Q 5/06** (2006.01)*

(86) International application number:
**PCT/JP2006/309982**

(87) International publication number:
**WO 2006/121214 (16.11.2006 Gazette 2006/46)**

(54) **PERMANENT WAVING AND SIMULTANEOUSLY COLORING AGENT**

DAUERHAFTE HAARVERFORMUNG UND GLEICHZEITIGE HAARFÄRBUNG

DEFORMATION PERMANENTE ET COLORATION SIMULTANEE

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.05.2005 JP 2005139808**

(43) Date of publication of application:
**12.03.2008 Bulletin 2008/11**

(73) Proprietor: **Showa Denko K.K.**
**Tokyo 105-8518 (JP)**

(72) Inventors:
- **SHIBUYA, Akira**
  **Kawasaki-shi, Kanagawa 2100865 (JP)**
- **KAMACHI, Motoaki**
  **Kawasaki-shi, Kanagawa 2100867 (JP)**

(74) Representative: **Strehl Schübel-Hopf & Partner**
**Maximilianstrasse 54**
**80538 München (DE)**

(56) References cited:
**GB-A- 721 831** **US-A- 2 577 710**
**US-A- 3 328 415** **US-A1- 2003 084 518**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 896 139 B1

## Description

FIELD OF THE INVENTION

The present invention relates to a permanent waving agent containing a cyclic mercapto compound. More particularly, the invention is concerned with a permanent waving agent capable of permanent waving and coloring hair simultaneously.

BACKGROUND OF THE INVENTION

Permanent waving agents are used by people of all ages for adding semipermanent curls and waves to hair. It is known that permanent waves are created through two steps, i.e., reductive splitting of cystine (disulfide) bonds of hair by the action of a reducing agent, and subsequent neutralization or fixing with an oxidizing agent, whereby the cystine (disulfide) bonds are restored.

The permanent waving of hair has generally used the so-called keratin reducing substances such as thioglycolic acid, cysteine, acetylcysteine and salts thereof. These keratin reducing substances exhibit a practical performance for permanent hair processing under alkaline conditions, and therefore most permanent solutions are adjusted to an alkaline pH of about 9.5. However, the alkaline permanent solutions are known to damage the hair and scalp. To solve such problems, permanent waving agents working in a neutral to weakly acidic pH range (pH: 3-7.5 at 25°C) have been developed.

Hairdyes now have widespread purposes, not only for elderly people to dye their white hair black, but also for young people to enjoy various colors of hair by bleaching their hair or by coloring their hair red or orange. Permanent hairdyes containing oxidation dyes are common. The demand is increasing of hair manicures with acid dyes that are safe and available in a wide choice of colors and provide bright results.

When both perming and coloring are desired, hair is generally permed first and then colored. Consequently, the treatments take a very long time, and hairdressers suffer a low turnover of customers. Such long treatments are also stressful for customers. In consideration of the pain on the customers, hair perming and coloring generally take place on different days. Thus, an effective method for simultaneously coloring and perming hair is desired.

The use of acid dyes at the permanent treatment has been attempted for simultaneously perming and manicuring hair in a shortened treatment time.

The conventional permanent waving method of hair with permanent waving agents uses a first agent containing a keratin reducing substance (reducing agent) (hereinafter, also referred to as the permanent waving first agent) and a second agent containing an oxidizing agent (hereinafter, also referred to as the permanent waving second agent). It has been proposed that a dye be added to the permanent waving second agent to effect coloring at the time of oxidation.

For example, JP-A-H09-151121 (Patent Document 1) discloses a straight perming second agent including an acid dye and an oxidizing agent. JP-A-2002-187822 (Patent Document 2) proposes a permanent waving second agent including hydrogen peroxide in which an acid dye is added and which has a pH of 2 to less than 4. JP-A-2004-123618 (Patent Document 3) discloses a hair cosmetic including three types of agents: agent A containing at least one compound selected from thioglycolic acid and salts thereof, cysteine and salts thereof, N-acetyl-L-cysteine and cysteamine; agent B-1 containing a direct dye and/or an oxidation dye; and agent B-2 containing an oxidizing agent. Patent Document 3 also proposes that the agent A be applied to hair followed by rinsing; and a liquid mixture of the agents B-1 and B-2 be applied to the hair.

Patent Documents 1 to 3 thus disclose incorporation of dyes in the permanent waving second agents.

Permanent waving first agents with dyes are also attempted. For example, GB Patent No. 721831 (Patent Document 4) discloses a permanent waving first agent including a mercapto compound such as thioglycolic acid, and an acid dye.

Patent Document 1: JP-A-H09-151121
Patent Document 2: JP-A-2002-187822
Patent Document 3: JP-A-2004-123618
Patent Document 4: GB Patent No. 721831

DISCLOSURE OF THE INVENTION

The second agents containing dyes as disclosed in Patent Documents 1 to 3 possess problems that the oxidizing agents denature the dyes and coloring results are unsatisfactory. Consequently, the second agents have to be left on hair for an extended period of time to penetrate into hair, leading to serious damage to the hair and skin.

The first agents used under alkaline condition containing acid dyes as disclosed in Patent Document 4 do not

produce satisfactory coloring results because the acid dyes, due to their electric charges, show good dyeing properties under acidic conditions, particularly at a pH less than 4, but the mercapto compounds as conventional reducing agent generally exhibit permanent waving performance under alkaline conditions. Further, the permanent waving first agents which work at an alkaline pH are too harsh to use on skin.

As described above, the conventional permanent waving agents have been unable to permanently wave and color hair at the same time. Consequently, the treatments take a very long time; for example, hair is permanent waved and on a later day is colored. This is a pain to the customers, and hairdressers suffer a low turnover of customers.

Thus, simultaneous permanent waving and coloring will reduce the burden on both hairdresser and customers and enable efficient treatments, and will be of great technical value.

The present inventors studied diligently and have found that the use of a cyclic mercapto compound represented by Formula (1) below as a keratin reducing substance leads to a higher perming performance in a neutral to weakly acidic pH range than is achieved by the known compounds. It has been also found that the use of the cyclic mercapto compound in combination with an acid dye or an oxidation dye enables simultaneous permanent waving and coloring in a neutral to weakly acidic pH range with little damage to the hair and skin. The present invention has been completed based on the findings.

The present invention is concerned with the following (1) to (9):

(1) A permanent waving agent comprising:

(i) a perming first agent including at least one compound represented by Formula (1) below or at least one compound selected from the group consisting of
N-(2-methoxy)ethyl-2-mercapto-4-butyrolactam,
N-(2-ethoxy)ethyl-2-mercapto-4-butyrolactam,
N- (2-methoxy)ethyl-2-mercapto-5-valerolactam, and
N-(2-ethoxy)ethyl-2-mercapto-5-valerolactam, and an acid dye and/or an oxidation dye:

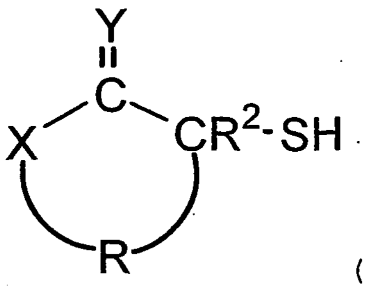

(1)

wherein X is a structure selected from the group consisting of -O-, -S-, -NH- and $-NR^1-$; $R^1$ is an alkyl group of 1 to 6 carbon atoms; $R^2$ is a hydrogen atom or an alkyl group of 1 to 6 carbon atoms; Y is an oxygen atom or a sulfur atom; and R is a divalent organic residue optionally having a mercapto group;
and
(ii) a perming second agent including an oxidizing agent.

(2) The permanent waving agent as described in (1), wherein X in Formula (1) is -O-, -NH-, $-NCH_3-$ or -S-.
(3) The permanent waving agent as described in (1) or (2), wherein Y in Formula (1) is an oxygen atom.
(4) The permanent waving agent as described in any one of (1) to (3), wherein R in Formula (1) is an alkylene group of 2 to 6 carbon atoms.
(5) The permanent waving agent as described in any one of (1) to (4), wherein R in Formula (1) is an alkylene group of 2 to 6 carbon atoms substituted with at least one mercapto group.
(6) The permanent waving agent as described in any one of (1) to (5), wherein the compound represented by Formula (1) is at least one compound selected from the group consisting of 2-mercapto-4-butyrolactone (another name:

2-mercapto-4-butanolide), 2-mercapto-4-butyrothiolactone,
2-mercapto-4-butyrolactam,
N-methyl-2-mercapto-4-butyrolactam,
N-ethyl-2-mercapto-4-butyrolactam,
2-mercapto-4-methyl-4-butyrolactone,

2-mercapto-4-ethyl-4-butyrolactone,
2-mercapto-5-valerolactone, 2-mercapto-5-valerolactam,
N-methyl-2-mercapto-5-valerolactam,
N-ethyl-2-mercapto-5-valerolactam, and
2-mercapto-6-hexanolactam.

(7) The permanent waving agent as described in any one of (1) to (6), wherein the perming first agent contains the compound represented by Formula (1) in an amount of 0.2 to 30% by mass in terms of thioglycolic acid reducing power.
(8) The permanent waving agent as described in any one of (1) to (7), wherein the perming first agent contains the acid dye and has a pH of 3.0 to 6.5.
(9) The permanent waving agent as described in any one of (1) to (7), wherein the perming first agent contains the oxidation dye and has a pH of 5.5 to 7.5.
(10) A method for permanent waving hair, using the permanent waving agent as described in any one of (1) to (9).

The cyclic mercapto compound used in the permanent waving agent according to the present invention has superior permanent waving practical performance and hair shaping/relaxing performance in a neutral to weakly acidic pH range. The use of the cyclic mercapto compound in combination with an acid dye or an oxidation dye enables simultaneous permanent waving and coloring at a pH level at which the oxidation dye or acid dye functions effectively, leading to high coloring effects.
Because the permanent waving and coloring are possible in a neutral to weakly acidic pH range, the treatment involves less irritation to the skin. The simultaneous permanent waving and coloring complete in a shorter period of time than ever, whereby the burden on both hairdresser and customers is reduced and the efficiency improved, leading to a high turnover of hairdresser customers.

PREFERRED EMBODIMENTS OF THE INVENTION

The present invention will be described in detail hereinbelow.
The permanent waving agent according to the present invention comprises a perming first agent including a cyclic mercapto compound as reducing agent, and an acid dye and/or an oxidation dye; and a perming second agent including an oxidizing agent.

[Perming first agent]

<u>Cyclic mercapto compound</u>

The cyclic mercapto compound in the first agent is represented by Formula (1) below:

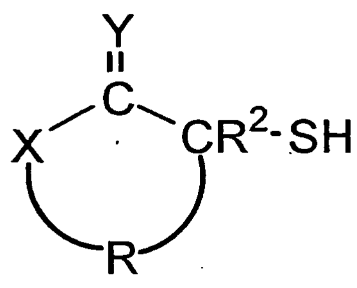

(1)

wherein X is a structure selected from the group consisting of -O-, -S-, -NH- and -$NR^1$-; $R^1$ is an alkyl group of 1 to 6 carbon atoms; $R^2$ is a hydrogen atom or an alkyl group of 1 to 6 carbon atoms; Y is an oxygen atom or a sulfur atom; and R is a divalent organic residue optionally having a mercapto group.
X is preferably -O-, -NH-, -$NCH_3$- or -S- in view of preparation of a perm solution, in which case the compound shows relatively high solubility in the perm aqueous solution.
Y is an oxygen atom or a sulfur atom, and is more preferably an oxygen atom in view of industrial availability and handling properties.
$R^2$ may be a hydrogen atom, a methyl group, an ethyl group or a propyl group, and is preferably a hydrogen atom, a methyl group or an ethyl group.
R is a divalent organic residue optionally having a mercapto (-SH) groups. R is not particularly limited as long

as it is a divalent organic group, and is preferably an alkylene group. The main chain of the alkylene group preferably has 2 to 6 carbon atoms. The divalent organic residue may have a branch or a side chain. Examples of the side chains include alkyl groups and alkenyl groups.

Where R is substituted with a mercapto group, one or more mercapto groups may be present.

Preferable examples of R include ethylene and propylene groups because of easy industrial availability.

Specific examples of the compounds represented by Formula (1) include 2-mercapto-3-propiolactone,
2-mercapto-2-methyl-3-propiolactone,
2-mercapto-3-methyl-3-propiolactone,
2-mercapto-3-ethyl-3-propiolactone,
2-mercapto-2,3-dimethyl-3-propiolactone,
2-mercapto-3-propiolactam,
2-mercapto-2-methyl-3-propiolactam,
2-mercapto-3-methyl-3-propiolactam,
2-mercapto-3-ethyl-3-propiolactam,
2-mercapto-2,3-dimethyl-3-propiolactam,
2-mercapto-3-propiothiolactone,
2-mercapto-2-methyl-3-propiothiolactone,
2-mercapto-3-methyl-3-propiothiolactone,
2-mercapto-3-ethyl-3-propiothiolactone,
2-mercapto-2,3-dimethyl-3-propiothiolactone,
2-mercapto-4-butyrolactone (another name:
2-mercapto-4-butanolide),
2-mercapto-2-methyl-4,4-dimethyl-4-butyrolactone,
2-mercapto-3-(2-propenyl)-4-butyrolactone,
2-mercapto-4-methyl-4-butyrolactone,
2-mercapto-2-methyl-4-butyrolactone,
2-mercapto-3-methyl-4-butyrolactone,
2-mercapto-4-methyl-4-butyrolactone,
2-mercapto-3,4-dimethyl-4-butyrolactone,
2-mercapto-2-ethyl-4-butyrolactone,
2-mercapto-3-ethyl-4-butyrolactone,
2-mercapto-4-ethyl-4-butyrolactone,
2-mercapto-4-butyrothiolactone,
2-mercapto-2-methyl-4-butyrothiolactone,
2-mercapto-3-methyl-4-butyrothiolactone,
2-mercapto-4-methyl-4-butyrothiolactone,
2-mercapto-3,4-dimethyl-4-butyrothiolactone,
2-mercapto-2-ethyl-4-butyrothiolactone,
2-mercapto-3-ethyl-4-butyrothiolactone,
2-mercapto-4-ethyl-4-butyrothiolactone,
2-mercapto-4-butyrolactam,
2-mercapto-2-methyl-4-butyrolactam,
2-mercapto-3-methyl-4-butyrolactam,
2-mercapto-4-methyl-4-butyrolactam,
2-mercapto-3,4-dimethyl-4-butyrolactam,
2-mercapto-2-ethyl-4-butyrolactam,
2-mercapto-3-ethyl-4-butyrolactam,
2-mercapto-4-ethyl-4-butyrolactam,
2-mercapto-5-valerolactone,
2-mercapto-2-methyl-5-valerolactone,
2-mercapto-3-methyl-5-valerolactone,
2-mercapto-4-methyl-5-valerolactone,
2-mercapto-5-methyl-5-valerolactone,
2-mercapto-2-ethyl-5-valerolactone,
2-mercapto-3-ethyl-5-valerolactone,
2-mercapto-4-ethyl-5-valerolactone,
2-mercapto-5-ethyl-5-valerolactone,
2-mercapto-5-valerolactam,

2-mercapto-2-methyl-5-valerolactam,
2-mercapto-3-methyl-5-valerolactam,
2-mercapto-4-methyl-5-valerolactam,
2-mercapto-5-methyl-5-valerolactam,
2-mercapto-2-ethyl-5-valerolactam,
2-mercapto-3-ethyl-5-valerolactam,
2-mercapto-4-ethyl-5-valerolactam,
2-mercapto-5-ethyl-5-valerolactam,
2-mercapto-5-valerothiolactone,
2-mercapto-2-methyl-5-valerothiolactone,
2-mercapto-3-methyl-5-valerothiolactone,
2-mercapto-4-methyl-5-valerothiolactone,
2-mercapto-5-methyl-5-valerothiolactone,
2-mercapto-2-ethyl-5-valerothiolactone,
2-mercapto-3-ethyl-5-valerothiolactone,
2-mercapto-4-ethyl-5-valerothiolactone,
2-mercapto-5-ethyl-5-valerothiolactone,
2-mercapto-6-hexanolactone,
2-mercapto-2-methyl-6-hexanolactone,
2-mercapto-3-methyl-6-hexanolactone,
2-mercapto-4-methyl-6-hexanolactone,
2-mercapto-5-methyl-6-hexanolactone,
2-mercapto-6-methyl-6-hexanolactone,
2-mercapto-6-hexanolactam,
2-mercapto-2-methyl-6-hexanolactam,
2-mercapto-3-methyl-6-hexanolactam,
2-mercapto-4-methyl-6-hexanolactam,
2-mercapto-5-methyl-6-hexanolactam,
2-mercapto-6-methyl-6-hexanolactam,
2-mercapto-6-hexanothiolactone,
2-mercapto-2-methyl-6-hexanothiolactone,
2-mercapto-3-methyl-6-hexanothiolactone,
2-mercapto-4-methyl-6-hexanothiolactone,
2-mercapto-5-methyl-6-hexanothiolactone,
2-mercapto-6-methyl-6-hexanothiolactone,
2-mercapto-7-heptanolactone, 2-mercapto-7-heptanothiolactone,
2-mercapto-7-heptanolactam, 2-mercapto-8-octanolactone,
2-mercapto-8-octanothiolactone, 2-mercapto-8-octanolactam,
2-mercapto-9-nonalactone, 2-mercapto-9-nonathiolactone,
2-mercapto-9-nonalactam, and N-methyl or N-ethyl derivatives of these lactams.

Of these, 2-mercapto-4-butyrolactone (another name:

2-mercapto-4-butanolide), 2-mercapto-4-butyrothiolactone,
2-mercapto-4-butyrolactam,
N-methyl-2-mercapto-4-butyrolactam,
N-ethyl-2-mercapto-4-butyrolactam,
2-mercapto-4-methyl-4-butyrolactone,
2-mercapto-4-ethyl-4-butyrolactone,
2-mercapto-5-valerolactone, 2-mercapto-5-valerolactam,
N-methyl-2-mercapto-5-valerolactam,
N-ethyl-2-mercapto-5-valerolactam, and
2-mercapto-6-hexanolactam and N-(2-methoxy)ethyl-2-mercapto-4-butyrolactam,
N-(2-ethoxy)ethyl-2-mercapto-4-butyrolactam,
N-(2-methoxy)ethyl-2-mercapto-5-valerolactam, and
N-(2-ethoxy)ethyl-2-mercapto-5-valerolactam are preferable in view of perming performance and industrial production. The permanent waving agent contains at least one such mercapto compound.

These compounds may be produced by known methods. For example, such compounds can be synthesized

by halogenating lactone compounds or lactam compounds followed by introduction of mercapto groups.

Mercaptolactones and mercaptothiolactones may be synthesized by a series of steps in which commercially available lactones or thiolactones are halogenated in accordance with a method described in J. Am. Chem. Soc. 1945,. 67. 2218-2220, and the synthesized halides or commercially available halides are produced into objective lactone derivatives by a method described in Ann. 1960, 639. 146-56.

Mercaptolactams may be synthesized by a series of steps in which halides are synthesized by a method described in J. Am. Chem. Soc. 1958. 80. 6233-6237, and the resultant halides are synthesized into objective lactam derivatives by a method described in Ann. 1960, 639. 146-56, similarly to the production of lactones.

The above mercapto compound is a keratin reducing substance and, when used as a main ingredient in perming agents, can work at a low pH that will not adversely affect the hair and can exhibit satisfactory permanent waving effects and hair relaxing and uncurling effects with little irritation to the skin. The reasons for these effects are not clearly understood but are believed to be that the structure of the compound provides higher lipophilicity than conventional reducing agents to enable increased penetration into hair and that because of having a heterocyclic ring, the mercapto compound is easily oxidized particularly under neutral to weakly acidic conditions, so that the hair processing agent can function as reducing agent without being rendered alkaline unlike with the conventional mercapto compounds.

The perming first agent may contain, in combination with the cyclic mercapto compound, one or more keratin reducing compounds selected from the group consisting of thioglycolic acid, thiolactic acid, cysteine, cysteamine, salts thereof, ester derivatives thereof and amide derivatives thereof.

Specific examples of the ester derivatives include N-acetylcysteine and acylcysteine, and further include esters of polyhydric alcohols as disclosed in JP-A-H08-291031. The polyhydric alcohols include 1,2-propanediol, 1,3-propanediol, 1-methoxypropanol(-2), 1-ethoxypropanol(-2), 1,3-butanediol, 1,4-butanediol, diethylene glycol and dipropylene glycol. These polyhydric alcohols may be glycol monoalkyl ethers such as monomethyl ethers and monoethyl ethers.

Specific examples of the amide derivatives include acetamide derivatives substituted with N-branched chain alkyls, and amide derivatives having hydroxyl groups or ether bonds as disclosed in JP-A-2000-507272 and JP-A-2003-528901.

The salts may be carboxylates or amine salts, and specific examples include ammonium thioglycolate, monoethanolamine thioglycolate and cysteine hydrochloride.

<u>Acid dyes and oxidation dyes</u>

The acid dyes contain sulfonic acid groups, carboxyl groups or the like. Various dyes are employable, including nitro dyes, azo dyes, nitroso dyes, triphenylmethane dyes, xanthene dyes, quinoline dyes, anthraquinone dyes and indigo dyes. Specific examples include red No. 2, red No. 3, red No. 102, red No. 104, red No. 105, red No. 106, yellow No. 4, yellow No. 5, green No. 3, blue No. 1, blue No. 2, red No. 201, red No. 227, red No. 230, red No. 231, red No. 232, orange No. 205, orange No. 207, yellow No. 202, yellow No. 203, green No. 201, green No. 204, green No. 205, blue No. 202, blue No. 203, blue No. 205, brown No. 201, red No. 401, red No. 502, red No. 503, red No. 504, red No. 506, orange No. 402, yellow No. 402, yellow No. 403, yellow No. 406, yellow No. 407, green No. 401, green No. 402, purple No. 401, black No. 401, acid black 52, acid blue 1, acid blue 3, acid blue 62, acid brown 13, acid green 50, acid orange 3, acid orange 6, acid red 14, acid red 35, acid red 73, acid red 184 and brilliant black 1. The acid dyes may be used singly or in combination of two or more kinds.

The oxidation dyes are compounds capable of producing colors by oxidation polymerization with the oxidizing agent in the second agent. The oxidation dyes are classified into precursors and couplers.

The precursors include para-phenylenediamine, toluene-2,5-diamine, 2-chloro-para-phenylenediamine, N-methoxyethyl-para-phenylenediamine, N,N-bis(2-hydroxyethyl)-para-phenylenediamine, 2-(2-hydroxyethyl)-para-phenylenediamine, 2,6-dimethyl-para-phenylenediamine, 4;4'-diaminodiphenylamine, 1,3-bis(N-(2-hydroxyethyl)-N-(4-aminophenyl)amino)-2-propan ol, PEG-3,3,2'-para-phenylenediamine, para-aminophenol, para-methylaminophenol, 3-methyl-4-aminophenol, 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-1-hydroxyethylpyrazole, 5-amino-ortho-cresol and salts thereof.

The couplers include metaphenylenediamine, 2,4-diaminophenoxyethanol, 2-amino-4-(2-hydroxyethylamino) anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, meta-aminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-meta-aminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, 3,3'-iminodiphenol, 2,4-diaminophenol, 1,4-diaminoanthraquinone, N,N-bis(4-aminophenyl)-2,5-diamino-1,4-quinodiimine, 5-(2-hydroxyethylamino)-2-methylphenol, N-phenyl-para-phenylenediamine and salts thereof.

Composition of first agent

The first agent contains the cyclic mercapto compound, and the acid dye or oxidation dye as essential components.

The agent preferably contains the cyclic mercapto compound of Formula (1) in an amount of 0.2 to 30% by mass, more preferably 1 to 15% by mass in terms of reducing power of thioglycolic acid. When the cyclic mercapto compound is contained in this amount, the agent used for permanent waving processing does not damage the hair and skin, and provides high waving efficiency.

When the content in terms of thioglycolic acid reducing power is less than 0.2% by mass, the agent often fails to function as hair processing agent. When the content exceeds 30% by mass, it is more likely that mongoloid hair, typical black hair, is excessively curled and the cuticle is partly removed, resulting in serious damage to the hair.

The content in terms of thioglycolic acid reducing power is a notation of keratin-reducing substance concentration specified with respect to each treatment in the Japanese permanent waving agent quality specification for medicated cosmetics, and is determined in accordance with the following process.

[Content in terms of thioglycolic acid reducing power]

Exactly 10 ml of a sample is placed in a 100-ml measuring flask, and purified water conforming with Japanese Standards of Cosmetic Ingredients (hereinafter, simply referred to as "water") is added to make the total volume 100 ml. The solution obtained is used as a test solution.

Exactly 20 ml of the test solution is mixed with 50 ml and 5 ml of water and 30% sulfuric acid, respectively, and the mixture is heated gently and boiled for 5 minutes. After cooling, the solution is titrated with 0.1N iodine solution, and the consumption A ml is obtained (indicator: starch test solution 3 ml). The titration result is put in the following formula to calculate the content in terms of thioglycolic acid:

$$\text{Reducing substance content (in terms of thioglycolic acid) (\% by mass)} = 0.4606 \times A$$

Japan Permanent Waving Lotion Industry Association imposes a voluntary control on permanent waving (curling) agents classified in cosmetics to usage calculated in a manner similar to the above.

When the cyclic mercapto compound is used together with other keratin reducing substances such as thioglycolic acid and thiolactic acid, these are preferably mixed in amounts such that the perming agent prepared has an analytical value of total reducing power within the above range.

The first agent preferably contains the acid dye or oxidation dye in an amount of 0. 01 to 15% by mass, more preferably 0.1 to 13% by mass, particularly preferably 0.2 to 10% by mass.

Formulations of the first agent of the invention are not particularly limited as long as the agent contains the cyclic mercapto compound represented by Formula (1) and the acid dye or oxidation dye. Examples of the formulations include liquids, foams, gels, creams and pastes. Depending on the formulation, the agent may be used as various types, including liquid type, spray type, aerosol type, cream type and gel type.

The first agent may contain other components for the purpose of improving the hair processing performance and depending on the usage formulation.

Such components include diluting agents and solvents. Examples of the preferable diluting agents and solvents in terms of versatility include water, dipropylene glycol, propylene glycol, glycerin, isopropanol, butanol, ethanol and 3-methoxy-3-propanol. Also suitable are polybutylene glycol 3-PEG/PPG-8/5 glycerin, polyethylene glycol (average molecular weight: 100-900), polypropylene glycol (average molecular weight: 130-1200), polyoxyethylene glyceryl ether (polymerization degree of ethylene oxide: 2-20) and polyoxypropylene glyceryl ether (polymerization degree of propylene oxide: 2-20).

Specific examples of the above include WILBRIDE S-753, PEG-400, PEG-600, PEG-800, NEWPOL PP-400, PRESTOL MGE-9, PRESTOL MGE-12 and PRESTOL MGP-9.

The first agent of the invention may contain other active components such as ultraviolet absorbers and hair protecting agents.

The cyclic mercapto compound is generally oily. The dissolution of the cyclic mercapto compound in water is slow when the compound is used in the form of aqueous solution which is a typical form of permanent waving agents. Therefore, two-phase separation often results. To solve such problems, the first agent may contain a surfactant.

The surfactant used herein may be anionic, cationic, amphoteric or nonionic, or may be a silicone surfactant or a biosurfactant.

The surfactants may be conventional.

The nonionic surfactants include:

polyoxyethylene alkyl ethers such as polyoxyethylene (2) lauryl ether, polyoxyethylene (4.2) lauryl ether, polyoxyethylene (9) lauryl ether, polyoxyethylene (21) lauryl ether, polyoxyethylene (23) lauryl ether, polyoxyethylene (25) lauryl ether, polyoxyethylene (2) cetyl ether, polyoxyethylene (5,5) cetyl ether, polyoxyethylene (7) cetyl ether, polyoxyethylene (10) cetyl ether, polyoxyethylene (15) cetyl ether, polyoxyethylene (20) cetyl ether, polyoxyethylene (23) cetyl ether, polyoxyethylene (25) cetyl ether, polyoxyethylene (30) cetyl ether, polyoxyethylene (40) cetyl ether, polyoxyethylene (2) stearyl ether, polyoxyethylene (4) stearyl ether, polyoxyethylene (20) stearyl ether, polyoxyethylene (5) behenyl ether, polyoxyethylene (10) behenyl ether, polyoxyethylene (20) behenyl esther, polyoxyethylene (30) behenyl ether, polyoxyethylene (2) alkyl ether, polyoxyethylene (4) alkyl ether and polyoxyethylene (10) alkyl ether;

polyoxyethylene alkylphenyl ethers such as polyoxyethylene (16) nonylphenyl ether;

polyoxyethylene alkenyl ethers such as polyoxyethylene (7) oleyl ether, polyoxyethylene (10) oleyl ether, polyoxyethylene (15) oleyl ether, polyoxyethylene (20) oleyl ether and polyoxyethylene (50) oleyl ether;

polyoxyethylene polyoxypropylene alkyl ethers such as polyoxyethylene (1) polyoxypropylene (4) cetyl ether, polyoxyethylene (10) polyoxypropylene (4) cetyl ether and polyoxyethylene (1) polyoxypropylene (8) cetyl ether;

polyoxyethylene alkyl alcohols such as polyoxyethylene (5) lanolin alcohol, polyoxyethylene (10) lanolin alcohol and polyoxyethylene (20) lanolin alcohol;

polyoxyethylene polyoxypropylene glycols;

polyoxyethylene polyoxypropylene alkyl glycols;

polyoxyethylene glyceryls such as polyoxyethylene (5) glyceryl monooleate and polyoxyethylene (15) glyceryl monooleate;

polyoxyethylenated castor oils such as polyoxyethylene (3) castor oil, polyoxyethylene (10) castor oil, polyoxyethylene (20) castor oil, polyoxyethylene (40) castor oil, polyoxyethylene (50) castor oil, polyoxyethylene (60) castor oil, polyoxyethylene (5) hydrogenerated castor oil, polyoxyethylene (10) hydrogenerated castor oil, polyoxyethylene (20) hydrogenerated castor oil, polyoxyethylene (30) hydrogenerated castor oil, polyoxyethylene (40) hydrogenerated castor oil, polyoxyethylene (50) hydrogenerated castor oil, polyoxyethylene (60) hydrogenerated castor oil and polyoxyethylene (80) hydrogenerated castor oil;

polyoxyethylene sorbitan fatty acid esters such as polyoxyethylene (6) sorbit monolaurate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan monoisostearate, polyoxyethylene (20) sorbitan tristearate, polyoxyethylene (6) sorbitan monooleate, polyoxyethylene (20) sorbitan monooleate, polyoxyethylene (20) sorbitan trioleate, polyoxyethylene (20) sorbitan coconut fatty acid ester, polyoxyethylene (10-80) sorbitan monolaurate, polyoxyethylene sorbitan tristearate, polyoxyethylene (20) sorbitan isostearate, polyoxyethylene (150) sorbitan tristearate, polyoxyethylene (6) sorbit tetraoleate, polyoxyethylene (30) sorbit tetraoleate, polyoxyethylene (40) sorbit tetraoleate and polyoxyethylene (60) sorbit tetraoleate;

sorbitan fatty acid esters such as sorbitan monolaurate, sorbitan monopalmitate, sorbitan monostearate, sorbitan monoisostearate, sorbitan monooleate, sorbitan sesquistearate, sorbitan sesquiisostearate, sorbitan sesquioleate, sorbitan tristearate, sorbitan trioleate, sorbitan coconut fatty acid ester, sorbitan isostearate, sorbitan sesquiisostearate and sorbitan distearate;

polyhydric alcohol fatty acid partial esters;

polyoxyethylene polyhydric alcohol fatty acid partial esters;

polyoxyethylene fatty acid monoesters (diesters);

polyglycerine fatty acid esters;

polyoxyethylene fatty acid amides such as polyoxyethylene (5) oleic acid amide;

fatty acid diethanol amides;

polyoxyethylene alkylamines;

triethanolamine fatty acid partial esters;

trialkylamine oxides; and

silicone nonionic surfactants such as polyoxyethylene/methylpolysiloxane copolymer (dimethicone copolyol) and

aminoethylaminopropylsiloxane/dimethylsiloxane copolymer (amodimethicone).

These surfactants may be used singly or in combination of two or more kinds.

In view of high emulsifying effect and easy handling, the present invention preferably uses at least one surfactant selected from the group consisting of the polyoxyethylene alkyl ethers, polyoxyethylene alkenyl ethers and polyoxyethylene alkylphenyl ethers containing 10 to 100 moles of polyoxyethylene added.

The silicone nonionic surfactants are also preferable in that hair lubrication and antistatic effects are expected. The silicone nonionic surfactants are known as polyether-modified silicone oils and amino-modified silicone oils, but the present invention classifies them as surfactants because they behave as nonionic surfactants. Commercially available nonionic surfactants include SH3771M (manufactured by Toray Dow Corning Silicone) and SM8704C (manufactured by Toray Dow Corning Silicone).

The cationic surfactants include cetyltrimethylammonium chloride, stearyltrimethylammonium chloride, lauryltrimethylammonium chloride, behenyltrimethylammonium chloride, distearyldimethylammonium chloride, dicocoyldimethylammonium chloride, dimethyldiallylammonium chloride/acrylic acid copolymer, dimethyldiallylammonium chloride/acrylic acid/acrylamide terpolymer, benzalkonium chloride, cetylpyridinium chloride, benzethonium chloride, lanolin-derived quaternary ammonium, amidoamines, dimethylaminopropylamide stearate, diethylaminoethylamide stearate, and silicone cationic surfactants (silanes and siloxanes having quaternary ammonium groups in the molecule) disclosed in JP-A-2004-176070.

The anionic surfactants include fatty acid salts; polyoxyethylenealkylethermethylcarboxylic acid salts; alkylbenzenesulfonic acid salts; alkylnaphthalenesulfonic acid salts; alkylsulfonic acid salts; α-olefinsulfonic acid salts; naphthalenesulfonic acid salt-formalin condensates; dialkyl sulfosuccinates such as dioctylsodium sulfosuccinate; disodiumalkylamidoethyl sulfosuccinates; α-sulfonated aliphatic alkyl ester salts; sodiumalkyl isethionates; petroleum sulfonic acid salts; alkylsulfonic acid salts such as sodium lauryl sulfate; alkyl ether sulfates such as ammonium lauryl ether sulfate; sulfated fats and oils; polyoxyethylene alkylsulfuric acid salts such as sodium polyoxyethylene (2,5) laurylsulfate; polyoxyethylene alkyl ether sulfuric acid salts; polyoxyethylene alkyl phenyl ether sulfuric acid salts; polyoxyethylene styrenated phenyl ether sulfuric acid salts; alkylphosphoric acid salts; polyoxyethylene alkyl ether phosphoric acid salts; polyoxyethylene alkyl phenyl ether phosphoric acid salts; and N-acyl-N-methyltaurine salts such as sodium N-methyl-N-oleyltaurine.

The amphoteric surfactants include:

carboxybetaines such as
N,N-dimethyl-N-alkyl-N-carboxymethyl ammonium betaine, N,N,N-trimethyl-N-alkylene ammonium carboxybetaine, betaine lauryldimethylaminoacetate and betaine coconut fatty acid amidopropyldimethylaminoacetate;
sulfobetaines such as
N-acylamidopropyl-N',N'-dimethyl-N'-β-hydroxypropylene ammonium sulfobetaine;
N,N-dialkyl-N,N-bis(polyoxyethylene sulfuric acid) ammonium betaines;
imidazolinium betaines such as
2-alkyl-1-hydroxyethyl-1-carboxymethyl imidazolinium betaine; and
N-coconut fatty acid
acyl-N-carboxymethyl-N-hydroxyethylethylenediaminesodium and N-coconut fatty acid
acyl-N-carboxymethyl-N-hydroxyethylethylenediamine/sodium laurylsulfate.

The polymeric surfactants include acrylic acid/methacrylic acid copolymer, polyacrylamide and sodium polyacrylate.

The biosurfactants include lecithins, hydrogenated lecithins, saponins, surfactins and/or salts thereof. As used herein, the biosurfactant means a substance synthesized by prokaryotes in their life activities and having properties similar to surfactants. Of such surfactants, the surfactins are compounds with a lipopeptide structure represented by Formula (3) below and/or their analogous compounds, or are compositions containing two or more kinds of such compounds.

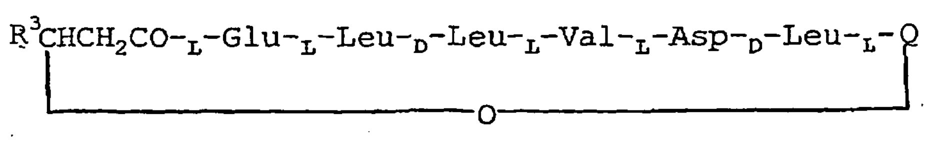

(3)

In Formula (3), Q is an amino acid residue selected from the group consisting of leucine, isoleucine, valine, glycine, serine, alanine, threonine, asparagine, glutamine, asparaginic acid, glutamic acid, lysine, arginine, cysteine, methionine, phenylalanine, tyrosine, tryptophan, histidine, proline, 4-hydroxyproline and homoserine, with leucine, isoleucine and valine being preferable.

$R^3$ is a normal alkyl group of 8 to 14 carbon atoms, an isoalkyl group of 8 to 14 carbon atoms, or an anteiso alkyl group of 8 to 14 carbon atoms. The normal alkyl group is a liner alkyl group, the isoalkyl group is generally $(CH_3)_2CH-(CH_2)_n-$, and the anteiso alkyl group is generally $CH_3-CH_2-CH(CH_3)-(CH_2)_n-$.

The analogous compounds may be such that part of the amino acids in Formula (3) is substituted with other amino acids. Specific examples include, but are not limited to, compounds in which the second L-leucine, fourth L-valine and sixth D-leucine are substituted with other amino acids.

The surfactins are generally produced by prokaryotes, and synthesized surfactins such as by chemical processes may be used. The prokaryotes generally used are Bacillus organisms such as Bacillus subtilis IAM 1213, IAM 1069, IAM 1259, IAM 1260, IFO 3035 and ATCC 21332.

The surfactin salts include alkali metal salts such as sodium, potassium and lithium salts; alkaline earth metal salts such as calcium and magnesium salts; and organic salts such as trimethylamine, triethylamine, tributylamine, monoethanolamine, diethanolamine, triethanolamine, lysine, arginine and choline salts. Of these, sodium salts of surfactins include surfactin sodium Aminofect™ commercially available from SHOWA DENKO K.K.

When the surfactant is used, a hydrophobic compound can be easily and uniformly dispersed in the diluting agent and solvent. The hydrophobic compounds include some perfumes contained in, for example, hydrocarbons. The emulsion obtained with use of the surfactant is uniform and is resistant to separation.

The amount of the surfactant may be determined appropriately depending on the purpose of use and viscosity of the solution, and is generally in the range of 0.01 to 20.0 parts by mass, preferably 0.02 to 15.0 parts by mass with respect to the cyclic mercapto compound 100 parts by mass.

The first agent may contain one or more perfumes for odor masking as required. Examples of the perfumes include (A) hydrocarbons, (B) alcohols, (C) phenols, (D) aldehydes and/or acetals, (E) ketones and/or ketals, (F) ethers, (G) synthetic musks, (H) acids, (1) lactones, (J) esters, (K) nitrogen-containing and/or sulfur-containing and/or halogen-containing compounds, and (L) natural perfumes. Specific examples of the perfumes include those disclosed in JP-A-2003-137758. The perfumes can mask the unpleasant odor of the mercapto compound and ammonia. A plurality of the perfumes may be used in combination to suit purposes and customer tastes. The content of the perfumes is not particularly limited as long as the unpleasant odor can be masked, but is generally in the range of 0.01 to 50 parts by mass, preferably 0.02 to 40 parts by mass with respect to the cyclic mercapto compound 100 parts by mass. When two or more perfumes are used in combination, the total content will be suitably in the above range.

The agent may further contain various additives such as swelling agents, penetration enhancers, buffers, lubricants, thickeners, hair protecting agents, wetting agents and stabilizers.

The swelling agents and penetration enhancers include ethanol, propanol, isopropanol, 1,2-propylene glycol, 1,3-butanediol, glycerol, ethylcarbitol, benzyl alcohol, benzyloxyethanol, urea and 2-methylpyrrolidone.

The lubricants include paraffin, liquid paraffin, beeswax, squalane, jojoba oil, olive oil, ester oil, triglyceride, vaseline and lanolin.

The thickeners include carboxymethylcellulose, carboxyvinyl polymers, hydroxyethylcellulose, hydroxypropylcellulose, xanthan gum, carrageenan, alginic acid salts, pectin, tragacanth gum, higher alcohols such as lauryl alcohol, cetyl alcohol, stearyl alcohol, isostearyl alcohol, oleyl alcohol and behenyl alcohol, kaolin, fatty acids such as lauric acid, myristic acid, palmitic acid, stearic acid, behenic acid, oleic acid, undecylic acid and isostearic acid, and vaseline.

The hair protecting agents include collagen and keratin hydrolysates and derivatives thereof.

The wetting agents include glycerol, diglycerol, propylene glycol, dipropylene glycol, 1,3-butanediol, sorbitol, plant extracts, vitamins, hyaluronic acid salts and chondroitin sulfate.

The stabilizers for preventing excessive reduction include disulfides of reducing compounds and dithiodiglycolic acid.

Other additives include chelating agents such as edetic acid, metal salts thereof, glutamic tetraacetic acid, metal salts thereof, asparaginic tetraacetic acid, metal salts thereof, propyl diamine tetraacetic acid, and metal salts thereof.

When the perming first agent contains the acid dye, the pH of the agent is desirably adjusted to from 2.0 to 7.0, preferably from 3.0 to 6.5. The first agent having a pH less than 2.0 can cause severe hair damage and skin irritation, and at a pH above 7.0 can show poor dyeing properties. When the perming first agent contains the oxidation dye, the pH is preferably adjusted to from 4.0 to 8.4, more preferably from 5.5 to 7.5 in view of bleaching and dyeing properties in association with the oxidizing agent in the second agent. When the first agent contains the oxidation dye, the pH of the second agent is preferably adjusted to from 5.0 to 9.5, more preferably from 6.0 to 9.0, in which case the dye is prevented from discoloration and produces a color favorably. When the pH of the second agent is less than 5.0, the oxidation dye often produces a color in unfavorable hue. The pH above 9.5 leads to decomposition of the oxidizing

agent, often resulting in unfavorable color production and damaged hair.

Herein, the pH is of the final perming first or second agent containing all the components such as water and additives, as measured at room temperature (23°C).

The pH adjustment may use a pH adjuster and a buffer.

The pH adjusters include organic acids, inorganic acids, bases and salts thereof. Specific examples include hydrochloric acid; organic acids such as citric acid, malic acid, lactic acid, succinic acid, oxalic acid, acetic acid, carbonic acid, tartaric acid, fumaric acid, levulinic acid, maleic acid and phosphoric acid, and sodium salts of the acids; ammonia, diethanolamine, triethanolamine, sodium hydroxide, potassium hydroxide, sodium carbonate, sodium hydrogencarbonate, potassium carbonate and potassium hydrogencarbonate. These may be used in combination of two or more kinds.

The buffers include inorganic buffers, and buffers containing basic amino acids such as arginine and lysine.

From the viewpoints of enhanced penetration of the acid dye into hair and fixing, the content of the pH adjusters and buffers is preferable in the range of 0.1 to 20% by mass, more preferably 1 to 10% by mass, particularly preferably 2 to 7% by mass with respect to the perming first agent.

The agent having the above pH seldom causes irritation to the skin and does not damage the hair and scalp. The first agent containing the cyclic mercapto compound is capable of excellent permanent waving performance at a weakly acidic pH level that causes less irritation to the skin. At the above pH, the acid dye produces high dyeing effects and provides hair with a uniform and satisfactory color.

In the invention, the first agent may be prepared in a desired composition prior to use, or may be prepared on site by mixing the materials immediately before use. In the on-site preparation, a solution of the compound of Formula (1) and optional components may be mixed with a solution of the acid dye or oxidation dye to give a solution agent. Alternatively, the compound of Formula (1) may be previously diluted with the diluting agent and solvent, and may be mixed with the acid dye or oxidation dye and optional components. The on-site preparation can avoid the lowering of dyeing properties of the acid dye or oxidation dye. In the on-site preparation, two or more kinds of the acid dyes may be used in combination to produce a unique hue. In particular, the agent is desirably prepared on site when using the oxidation dye having an amino group.

In the on-site preparation, an acid or salt thereof may be added to any solution describes above, such that the agent finally obtained will have a pH in the aforesaid range.

[Perming second agent]

The perming second agent contains an oxidizing agent. Commercially available agents may be used without limitation.

Examples of the oxidizing agents include bromates, perborates, hydrogen peroxide and persulfates (such as ammonium persulfate, potassium persulfate and sodium persulfate). The amount of the oxidizing agents is about 3 to 10% by mass. This amount of the oxidizing agents results in high effects. Hydrogen peroxide and persulfates are suitable for use with the oxidation dye from the viewpoint of dyeing properties.

A pH adjuster may be used as required. Materials commonly used in perming second agents may be added while still achieving the effects of the invention. Such materials include fats and oils, higher alcohols, perfumes, hair protecting agents, polypeptides, ultraviolet absorbers, antiseptic agents, moisturizing agents, cationic polymers, anionic polymers and nonionic polymers.

Formulations of the perming second agent are not limited and include creams, gels, emulsions, transparent liquids and powders.

The permanent waving agent according to the present invention can produce permanent waves and color through a series of steps as follows. When the hair dyeing takes place depends on the dye used. In the case of the acid dye, the hair dyeing generally takes place during the processing with the first agent. With the oxidation dye, the hair dyeing proceeds while the cyclic mercapto compound is oxidized with the second agent.

[Permanent waving method]

The use of the permanent waving agent is not particularly limited. For example, it may be used for the permanent waving of hair as described below. In the invention, the permanent waving treatments include permanent waving treatment itself, permanent wave smoothing treatment and frizz straightening treatment.

(1) The perming first agent is applied to hair, and the wet hair is wound on rods for shaping. The hair may be first wetted with water, wound on rods, and then given the permanent first agent.
(2) The hair wet with the agent is allowed to stand at room temperature, preferably at elevated temperatures of about 30 to 40°C.
(3) The cyclic mercapto compound is oxidized with the perming second agent, and the hair is fixed.

(4) The rods are removed from the fixed hair, and the hair is rinsed, shampooed and dried.

The permanent waving agent has little adverse effects on the skin and low sensitizing potential, and enables superior waving efficiency and simultaneous coloring. Consequently, the agent can improve the efficiency and reduce the pain on the operators and customers.

According to the present invention, hair can be permed and colored in a weakly acidic pH range that causes less irritation to the hair and scalp. Because the heretofore separated perming and coloring can be performed simultaneously, the burden on both hairdresser and customers is reduced and the treatment efficiency is enhanced.

EXAMPLES

The present invention will be described with reference to the following examples, but it should be construed that the invention is in no way limited to the examples.

[Synthetic Example 1]

Production of 2-mercapto-4-butyrolactone

70% Sodium hydrosulfide (49 g, 0.6 mmol, manufactured by JUNSEI CHEMICAL CO., LTD.) was dissolved in a mixture of methyl alcohol (500 g, special grade, manufactured by JUNSEI CHEMICAL CO., LTD.) and purified water (500 g, water distilled and passed through an ion exchange filter). The resultant solution was cooled with ice to not more than 10°C with stirring. To the cooled solution, 2-bromo-4-butyrolactone (100 g, 0.6 mol, manufactured by Tokyo Kasei Kogyo Co., Ltd.) was added dropwise over a period of about 30 minutes. After the completion of the dropwise addition, the mixture was stirred for 10 minutes, and the reaction liquid was concentrated to approximately half of the original volume under reduced pressure.

To the concentrated liquid was added ethyl acetate (500 ml, special grade, manufactured by JUNSEI CHEMICAL CO., LTD.) followed by extraction. The aqueous phase obtained was subjected to re-extraction with ethyl acetate (500 ml). The organic phases thus extracted were combined and concentrated and purified by distillation under reduced pressure to give 2-mercapto-4-butyrolactone (23 g, bp: 94°C/0.3 kPa, yield: 32%).

[Examples 1 to 7]

Hair was permanent waved with perming first and second agents prepared as described below. The perming performance and resultant color were evaluated.

Preparation of perming first agent

Liquid A (reducing agent): 2.6 g of 2-mercapto-4-butyrolactone (MBL) placed in a 150-ml glass vessel with a lid.

Liquid B (diluting agent): A substantially uniform solution placed in a 100-ml polyethylene vessel with a lid, which consisted of 84.7 g of purified water , 0.3 g of an acid dye indicated in Table 1, 10 g of propylene glycol, 0.4 g of polyoxyethylene/methylpolysiloxane copolymer, and 2 g of polyoxyethylene (20) cetyl ether.

The lid of the vessel containing the liquid B was opened, and the vessel was turned upside down to pour the content into the vessel containing the liquid A. The lid of the vessel containing the mixture was closed, and the vessel was shaken for 30 seconds so that the liquids A and B became uniform. Consequently, a perming first agent was obtained.

Preparation of perming second agent

7 g of sodium bromate and 93 g of purified water were mixed together to give a perming second agent.

Permanent waving treatment

Hair of the treatment subject was shampooed, sufficiently rinsed, and towel dried. The hair was permanent waved and colored with the previously prepared first and second agents in the following manner.

(1) The perming first agent was applied to the hair, and the wet hair was wound on rods for shaping. The first agent was applied again in small amounts so that the hair was evenly wet with the agent.

(2) The head of the subject was covered with a haircap, and heat was applied with a heater so that the temperature in the haircap was about 40°C. The hair was heated at the temperature for 15 minutes.

(3) After 15 minutes, the haircap was removed. The hair wound on the rods was evenly wetted with the perming second agent.
(4) The hair wet with the perming second agent was allowed to stand for 15 minutes at room temperature.
(5) The rods were removed from the hair, and the hair was rinsed, shampooed, conditioned and dried.

The subject's hair was medium long, and the treatment (containing permanent waving and coloring) took 75 minutes.

[Example 8]

The procedures in Examples 1 to 7 were repeated, except that the acid dye in the perming first agent was black No. 401, and the treatment subject was a white-haired female.

[Comparative Example 1]

Hair was permanent waved with perming first and second agents prepared as described below. After the permanent waving, the permed hair was colored with a dyeing agent prepared as described below. The condition of hair was evaluated.

Preparation of perming first agent

Liquid A (reducing agent): 2.6 g of 2-mercapto-4-butyrolactone placed in a 150-ml glass vessel with a lid.
Liquid B (diluting agent): A substantially uniform solution placed in a 100-ml polyethylene vessel with a lid, which consisted of 85 g of purified water, 10 g of propylene glycol, 0.4 g of polyoxyethylene/methylpolysiloxane copolymer, and 2 g of polyoxyethylene (20) cetyl ether.
The lid of the vessel containing the liquid B was opened, and the vessel was turned upside down to pour the content into the vessel containing the liquid A. The lid of the vessel containing the mixture was closed, and the vessel was shaken for 30 seconds so that the liquids A and B became uniform. Consequently, a perming was added to make the total amount 100 g.

Permanent waving treatment and coloring treatment

Hair of the treatment subject was shampooed, sufficiently rinsed, and towel dried. The hair was permanent waved with the previously prepared first and second agents, and was subsequently colored with the dye agent in the following manner.

(1) The perming first agent was applied to the hair, and the wet hair was wound on rods for shaping. The first agent was applied again in small amounts so that the hair was evenly wet with the agent.
(2) The head of the subject was covered with a haircap, and heat was applied with a heater so that the temperature in the haircap was about 40°C. The hair was heated at the temperature for 15 minutes.
(3) After 15 minutes, the haircap was removed. The hair wound on the rods was evenly wetted with the perming second agent.
(4) The hair wet with the perming second agent was allowed to stand for 15 minutes at room temperature.
(5) The rods were removed from the hair, and the hair was rinsed, shampooed, conditioned and dried with a dryer.
(6) The dyeing agent was evenly applied to the permed hair.
(7) The dyeing agent was left on for 20 minutes.
(8) The hair was shampooed to wash away the excess agent, and was conditioned and dried with a drier.

The subject's hair was medium long, and the permanent waving and coloring treatments took 180 minutes.

[Comparative Example 2].

Hair was colored with the dyeing agent described in Comparative Example 1. After the coloring, the colored hair was permanent waved with the perming first and second agents described in Comparative Example 1. The condition of hair was evaluated.

Coloring treatment and permanent waving treatment

Hair of the treatment subject was shampooed, sufficiently rinsed, and towel dried. The hair was colored and subsequently permanent waved in the following manner.

(1) The dyeing agent was evenly applied to the hair.
(2) The dyeing agent was left on for 20 minutes.
(3) The hair was shampooed to wash away the excess agent, and was towel dried. The hair was substantially dry.
(4) The perming first agent was applied to the hair, and the wet hair was wound on rods for shaping. The first agent was applied again in small amounts so that the hair was evenly wet with the agent.
(5) The head of the subject was covered with a haircap, and heat was applied with a heater so that the temperature in the haircap was about 40°C. The hair was heated at the temperature for 15 minutes.
(6) After 15 minutes, the haircap was removed. The hair wound on the rods was evenly wetted with the perming second agent.
(7) The hair wet with the perming second agent was allowed to stand for 15 minutes at room temperature.
(8) The rods were removed from the hair, and the hair was rinsed, shampooed, conditioned and dried with a dryer.

The subject's hair was medium long, and the coloring and permanent waving treatments took 160 minutes.

[Comparative Example 3]

Hair was permanent waved with perming first and second agents prepared as described below, and the permed hair was colored with a dyeing agent prepared as described below. The permanent waving and coloring were performed as described in Comparative Example 1. The condition of hair was evaluated. The subject's hair was medium long, and the permanent waving and coloring treatments took 180 minutes.

Preparation of perming first agent

14 g of 50% ammonium thioglycolate aqueous solution, 5 g of 80% monoethanolamine aqueous solution, 4 g of polyoxyethylene (23) cetyl ether, 2 g of stearyltrimethylammonium chloride and 1 g of dimethylpolysiloxane were dissolved in 74 g of purified water. Consequently, a perming first agent was obtained.

Preparation of perming second agent

7 g of sodium bromate and 93 g of purified water were mixed together to give a perming second agent.

Dyeing agent

0.3 g of orange No. 205, 1.0 g of hydroxyethylcellulose, 8.0 g of benzyl alcohol, 25 g of ethanol and 3 g of citric acid were dissolved in 50 g of purified water. The solution was adjusted to pH 3.5 with 28% ammonia water, and purified water was added to make the total amount 100 g.

Evaluation of treated hair

The hair treated in Examples 1-8 and Comparative Examples 1-3 was visually evaluated for colors and curls.

Color:

AA: The color exhibition was equal to or better than that obtained using the same dye in accordance with Comparative Example 1.
BB: The color exhibition was rather inferior to that obtained using the same dye in accordance with Comparative Example 1.
CC: The color exhibition was clearly inferior to that obtained using the same dye in accordance with Comparative Example 1.

Curls:

AA: The curl creation was equal to or better than that obtained in Comparative Example 2.

BB: The curls were loose.
CC: Curls were not created.

Feel:

AA: The feel was equal to or better than the hair treated in Comparative Example 2.
CC: The feel was inferior to the hair treated in Comparative Example 2.

Table 1

| | Reducing agent | Dye | Treat Time (min) | ment Color | Curls | Feel |
|---|---|---|---|---|---|---|
| Ex.1 | MBL | Orange No. 205 | 75 | AA | AA | AA |
| Ex.2 | MBL | Purple No. 401 | 75 | AA | AA | AA |
| Ex.3 | MBL | Red No. 106 | 75 | AA | AA | AA |
| Ex.4 | MBL | Red No. 227 | 75 | AA | AA | AA |
| Ex.5 | MBL | Yellow No. 203 | 75 | AA | AA | AA |
| Ex.6 | MBL | Green No. 204 | 75 | AA | AA | AA |
| Ex.7 | MBL | Blue No. 202 | 75 | AA | AA | AA |
| Ex.8 | MBL | Black No. 401 | 75 | AA | AA | AA |
| Co.Ex.1 | MBL | Orange No. 205 | 180 | AA | BB | AA |
| Co.Ex.2 | MBL | Orange No. 205 | 160 | BB | AA | AA |
| Co.Ex.3 | Thioglyc olic acid | Orange No. 206 | 180 | AA | AHA | CC |

[Examples 9 and 10]

Preparation of perming first agent

Liquid A (reducing agent): 2.6 g of 2-mercapto-4-butyrolactone placed in a 150-ml glass vessel with a lid.
Liquid B (diluting agent): 97.4 g of a solution of a composition indicated in Table 2, placed in a 100-ml polyethylene vinyl chloride vessel with a lid.
The lid of the vessel containing the liquid B was opened, and the vessel was turned upside down to pour the content into the vessel containing the liquid A. The lid of the vessel containing the mixture was closed, and the vessel was shaken for 30 seconds so that the liquids A and B became uniform. Consequently, a perming first agent was obtained.

Preparation of perming second agent

17 g of 35% hydrogen peroxide aqueous solution and purified water were mixed together to give 100 g of a perming second agent.

Table 2

| Composition of liquid B for oxidation dyeing | |
|---|---|
| Ex. 9 | |
| Toluene-2,5-diamine | 0.3 g |
| Orthoaminophenol | 0.3 g |
| Resorcin | 0.2 g |
| POE (9) oleyl ether | 8 g |
| PEG 400 | 10 g |
| Stearyltrimethylammonium chloride | 2 g |
| Purified water | Balance |

(continued)

| Ex. 10 | |
|---|---|
| Para-phenylenediamine | 0.1 g |
| Resorcin | 0.1 g |
| Para-aminophenol | 0.2 g |
| Meta-aminophenol | 0.1 g |
| POE (9) oleyl ether | 8 g |
| PEG 400 | 10 g |
| Stearyltrimethylammonium chloride | 2 g |
| Purified water | Balance |

Evaluation of Examples 9 and 10

Hair of the treatment subject was shampooed, sufficiently rinsed, and towel dried. The hair was permanent waved and dyed with an oxidation dye in the following manner.

(1) The mixture (first agent) which was obtained by mixing the each liquid B in Table 2 with liquid A was evenly applied to the hair in a manner, respectively. On this occasion, an equal amount of the first agent was applied to each approximately 5 g of hair and the hair was combed to spread the agent evenly.
(2) The wet hair was wound relatively tightly on small rods (6 to 8 mm in inner diameter).
(3) The first agent was applied again to the wound hair, and the head of the subject was covered with a wrapping film. The hair was allowed to stand at an elevated temperature for about 20 minutes.
(4) The wrapping film was removed, and the second agent was applied.
(5) The wet hair was allowed to stand for 5 minutes.
(6) The second agent was applied two times every 5 minutes likewise in (4) and (5).
(7) After 20 minutes, the rods were removed from the hair, and the hair was rinsed, shampooed, conditioned and dried with a dryer.

The treated hair in each Example 9 and 10 showed curls and color that were comparable to those produced through conventional two steps of oxidation dyeing and perming.

## Claims

**1.** A permanent waving agent comprising:

(i) a perming first agent including at least one compound represented by Formula (1) below or at least one compound selected from the group consisting of
N-(2-methoxy)ethyl-2-mercapto-4-butyrolactam,
N-(2-ethoxy)ethyl-2-mercapto-4-butyrolactam,
N-(2-methoxy)ethyl-2-mercapto-5-valerolactam, and
N-(2-ethoxy)ethyl-2-mercapto-5-valerolactam
and an acid dye and/or an oxidation dye:

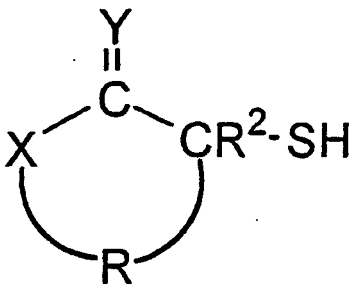

(1)

wherein X is a structure selected from the group consisting of -0-, -S-, -NH- and -$NR^1$-; $R^1$ is an alkyl group of 1 to 6 carbon atoms; $R^2$ is a hydrogen atom or an alkyl group of 1 to 6 carbon atoms; Y is an oxygen atom or a sulfur atom; and R is a divalent organic residue optionally having a mercapto group; and
(ii) a perming second agent including an oxidizing agent.

2. The permanent waving agent according to claim 1, wherein X in Formula (1) is -0-, -NH-, -$NCH_3$- or -S-.

3. The permanent waving agent according to claim 1, wherein Y in Formula (1) is an oxygen atom.

4. The permanent waving agent according to claim 1, wherein R in Formula (1) is an alkylene group of 2 to 6 carbon atoms.

5. The permanent waving agent according to claim 1, wherein R in Formula (1) is an alkylene group of 2 to 6 carbon atoms substituted with at least one mercapto group.

6. The permanent waving agent according to claim 1, wherein the compound represented by Formula (1) is at least one compound selected from the group consisting of
2-mercapto-4-butyrolactone (another name: 2-mercapto-4-butanolide),
2-mercapto-4-butyrothiolactone,
2-mercapto-4-butyrolactam,
N-methyl-2-mercapto-4-butyrolactam,
N-ethyl-2-mercapto-4-butyrolactam,
2-mercapto-4-methyl-4-butyrolactone,
2-mercapto-4-ethyl-4-butyrolactone,
2-mercapto-5-valerolactone,
2-mercapto-5-valerolactam,
N-methyl-2-mercapto-5-valerolactam,
N-ethyl-2-mercapto-5-valerolactam, and 2-mercapto-6-hexanolactam.

7. The permanent waving agent according to claim 1, wherein the perming first agent contains the compound represented by Formula (1) in an amount of 0.2 to 30% by mass in terms of thioglycolic acid reducing power.

8. The permanent waving agent according to claim 1, wherein the perming first agent contains the acid dye and has a pH of 3.0 to 6.5.

9. The permanent waving agent according to claim 1, wherein the perming first agent contains the oxidation dye and has a pH of 5.5 to 7.5.

10. A method for permanent waving hair, using the permanent waving agent as described in claim 1.

**Patentansprüche**

1. Mittel zur dauerhaften Haarverformung, welches umfasst:

(i) ein erstes Dauerwellenmittel, welches mindestens eine Verbindung der nachstehenden Formel (1) oder mindestens eine Verbindung, die aus der aus
N-(2-Methoxy)ethyl-2-mercapto-4-butyrolactam,
N-(2-Ethoxy)ethyl-2-mercapto-4-butyrolactam,
N-(2-Methoxy)ethyl-2-mercapto-5-valerolactam und
N-(2-Ethoxy)ethyl-2-mercapto-5-valerolactam bestehenden Gruppe ausgewählt ist, und einen Säurefarbstoff und/oder einen Oxidationsfarbstoff enthält:

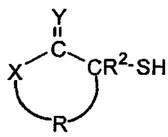

(1)

worin X eine Struktur ist, die aus der Gruppe ausgewählt ist, die aus -0-, -S-, -NH- und $-NR^1-$ besteht, wobei $R^1$ eine Alkylgruppe mit 1 bis 6 Kohlenstoffatomen ist; $R^2$ ein Wasserstoffatom oder eine Alkylgruppe mit 1 bis 6 Kohlenstoffatomen ist; Y ein Sauerstoffatom oder ein Schwefelatom ist; und R ein zweiwertiger organischer Rest ist, der gegebenenfalls eine Mercaptogruppe aufweist; und
(ii) ein zweites Dauerwellenmittel, das ein Oxidationsmittel enthält.

**2.** Mittel zur dauerhaften Haarverformung nach Anspruch 1, worin X in der Formel (1) -0-, -NH-, $-NCH_3-$ oder -S- ist.

**3.** Mittel zur dauerhaften Haarverformung nach Anspruch 1, worin Y in der Formel (1) ein Sauerstoffatom ist.

**4.** Mittel zur dauerhaften Haarverformung nach Anspruch 1, worin R in der Formel (1) eine Alkylengruppe mit 2 bis 6 Kohlenstoffatomen ist.

**5.** Mittel zur dauerhaften Haarverformung nach Anspruch 1, worin R in der Formel (1) eine Alkylengruppe mit 2 bis 6 Kohlenstoffatomen ist, die mit mindestens einer Mercaptogruppe substituiert ist.

**6.** Mittel zur dauerhaften Haarverformung nach Anspruch 1, worin die Verbindung der Formel (1) mindestens eine Verbindung ist, die aus de Gruppe ausgewählt ist, die aus
2-Mercapto-4-butyrolacton (anderer Name: 2-Mercapto-4-butanolid),
2-Mercapto-4-butyrothiolacton,
2-Mercapto-4-butyrolactam,
N-Methyl-2-mercapto-4-butyrolactam,
N-Ethy1-2-mercapto-4-butyrolactam,
2-Mercapto-4-methyl-4-butyrolacton,
2-Mercapto-4-ethyl-4-butyrolacton,
2-Mercapto-5-valerolacton,
2-Mercapto-5-valerolactam,
N-Methyl-2-mercapto-5-valerolactam,
N-Ethyl-2-mercapto-5-valerolactam,
2-Mercapto-6-hexanolactam
besteht.

**7.** Mittel zur dauerhaften Haarverformung nach Anspruch 1, wobei das erste Dauerwellenmittel die Verbindung der Formel (1) in einer Menge von 0,2 bis 30% Massen-%, ausgedrückt als Thioglycolsäure-Reduzierkraft, enthält.

**8.** Mittel zur dauerhaften Haarverformung nach Anspruch 1, wobei das erste Dauerwellenmittel den Säurefarbstoff enthält und einen pH-Wert von 3,0 bis 6,5 aufweist.

**9.** Mittel zur dauerhaften Haarverformung nach Anspruch 1, wobei das erste Dauerwellenmittel den Oxidationsfarbstoff enthält und einen pH-Wert von 5,5 bis 7,5 aufweist.

**10.** Verfahren zum dauerhaften Haarverformen, wobei das Mittel zur dauerhaften Haarverformung nach Anspruch 1

eingesetzt wird.

## Revendications

1. Agent d'ondulation permanente comprenant :

(i) un premier agent de réalisation de permanente comprenant au moins un composé représenté par la formule (1) ci-dessous ou au moins un composé sélectionné dans le groupe constitué de
N-(2-méthoxy)éthyl-2-mercapto-4-butyrolactame,
N-(2-éthoxy)éthyl-2-mercapto-4-butyrolactame,
N-(2-méthoxy)éthyl-2-mercapto-5-valérolactame, et
N-(2-éthoxy)éthyl-2-mercapto-5-valérolactame
et un colorant acide et/ou un colorant d'oxydation :

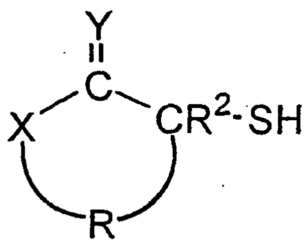

(1)

où X est une structure sélectionnée dans le groupe constitué de -O-, -S-, -NH- et -$NR^1$- ; $R^1$ est un groupe alkyle de 1 à 6 atomes de carbone ; $R^2$ est un atome d'hydrogène ou un groupe alkyle de 1 à 6 atomes de carbone ; Y est un atome d'oxygène ou un atome de soufre ; et R est un résidu organique divalent ayant facultativement un groupe mercapto ; et
(ii) un second agent de réalisation de permanente comprenant un agent oxydant.

2. Agent d'ondulation permanente selon la revendication 1, où X dans la formule (1) est -O-, -NH-, -$NCH_3$- ou -S-.

3. Agent d'ondulation permanente selon la revendication 1, où Y dans la formule (1) est un atome d'oxygène.

4. Agent d'ondulation permanente selon la revendication 1, où R dans la formule (1) est un groupe alkylène de 2 à 6 atomes de carbone.

5. Agent d'ondulation permanente selon la revendication 1, où R dans la formule (1) est un groupe alkylène de 2 à 6 atomes de carbone substitué par au moins un groupe mercapto.

6. Agent d'ondulation permanente selon la revendication 1, où le composé représenté par la formule (1) est au moins un composé sélectionné dans le groupe constitué de
2-mercapto-4-butyrolactone (autre dénomination : 2-mercapto-4-butanolide),
2-mercapto-4-butyrothiolactone,
2-mercapto-4-butyrolactame,
N-méthyl-2-mercapto-4-butyrolactame,
N-éthyl-2-mercapto-4-butyrolactame,
2-mercapto-4-méthyl-4-butyrolactone,
2-mercapto-4-éthyl-4-butyrolactone,
2-mercapto-5-valérolactone,
2-mercapto-5-valérolactame,
N-méthyl-2-mercapto-5-valérolactame,
N-éthyl-2-mercapto-5-valérolactame, et
2-mercapto-6-hexanolactame.

7. Agent d'ondulation permanente selon la revendication 1, où le premier agent de réalisation de permanente contient

le composé représenté par la formule (1) en une quantité de 0,2 à 30 % en masse en termes de pouvoir de réduction de l'acide thioglycolique.

**8.** Agent d'ondulation permanente selon la revendication 1, où le premier agent de réalisation de permanente contient le colorant acide et a un pH de 3,0 à 6,5.

**9.** Agent d'ondulation permanente selon la revendication 1, où le premier agent de réalisation de permanente contient le colorant d'oxydation et a un pH de 5,5 à 7,5.

**10.** Procédé d'ondulation permanente des cheveux, utilisant l'agent d'ondulation permanente selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP A A
- JP H09151121 B
- JP 2002187822 A
- JP 2004123618 A
- GB 721831 A
- JP H09151121 A
- JP H08291031 A
- JP 2000507272 A
- JP 2003528901 A
- JP 2004176070 A
- JP 2003137758 A


### Non-patent literature cited in the description


- *J. Am. Chem. Soc.,* 1945, vol. 67, 2218-2220
- *Ann.,* 1960, vol. 639, 146-56
- *J. Am. Chem. Soc.,* 1958, vol. 80, 6233-6237